# DEMANDE DE BREVET EUROPEEN

(11) **EP 2 540 259 A1**
(43) Date de publication de la demande: **02.01.2013**
(21) Numéro de dépôt: 12173328.1
(22) Date de dépôt: 25.06.2012
(51) Int. Cl.: A61F 5/01, A63B 71/12, A41D 13/015, A41D 13/06

(54) **Orthèse de genoux**

(30) Priorité: 30.06.2011 FR 1102041
(71) Demandeur: Desert, Cédric, 62162 Vieille Eglise (FR)
(72) Inventeur: Desert, Cédric, 62162 Vieille Eglise (FR)
(74) Mandataire: Bureau Duthoit Legros Associés

(57) **Abrégé**

L'invention est relative à une orthèse de genou comprenant :
- un cadre de jambe supérieur (1) destiné à maintenir la cuisse, dit coque de cuisse, et un cadre de jambe inférieur (2) destiné à maintenir le mollet, dit coque tibiale, ladite coque de cuisse et coque tibiale étant destinées à être positionnées au dessus et en dessous de la rotule du genou,
- des moyens d'articulation (5) reliant les cadres de jambe supérieur et inférieur (1,2), destinés à être positionnés latéralement au genou,
- une coque (9) de protection de la rotule, mobile par rapport auxdites coques de cuisse et tibiale, et destinée à être positionnée sur la rotule dudit genou, reliée auxdits moyens d'articulation (5) par l'intermédiaire de liaisons souples (11),

La présente invention comprend outre la coque (9) de protection de la rotule, dite première coque de protection, une seconde coque (12) de protection apte à couvrir le jour entre ladite coque (9) de protection de la rotule et ladite coque tibiale (2).

## Description

L'invention est relative à une orthèse de genou comprenant des moyens de protection de la rotule et trouvant une application particulière pour la pratique de sports tels que le motocross.

Le domaine de l'invention est celui des dispositifs orthopédiques.

De manière classique, les orthèses de genou comprennent des cadres de jambe supérieur et inférieur comportant des coussins adoptant la forme des cadres et venant se positionner sur la cuisse et le tibia. Ces deux cadres sont articulés entre eux au moyen d'articulations plus ou moins complexes, permettant de suivre le mouvement de pivotement du genou. Ce type d'orthèse apporte un soutien du genou lorsque ce dernier est fragilisé mais ne fournit pas de protection de la rotule contre un choc éventuel.

Il a déjà été proposé de mettre en place une coque de protection de la rotule fixée au niveau des articulations pour protéger la rotule d'un choc éventuel. Cependant, ce type de protection ne fournit pas une protection fiable lors d'une pratique sportive comme le motocross, un jour étant présent entre le cadre de jambe inférieur et la coque de protection de la rotule. De tels dispositifs de protection du genou ne fournissent pas une protection fiable quelque soit le degré de fléchissement de la jambe de l'utilisateur.

Il existe ainsi un certain nombre d'orthèses de genou comprenant une protection de la rotule.

On connait ainsi de l'état de la technique une orthèse de genou comprenant une protection de la rotule capable d'absorber les chocs, par exemple le document US D548 844.

Dans ce document, la technique pour protéger la rotule du genou consiste à assujettir une coque de protection de la rotule aux articulations au moyen de liaisons souples de manière à amortir un choc. Deux autres protections sont disposées au dessus et en dessous de la coque de protection de la rotule de manière à couvrir les jours entre la coque de protection de la rotule et les cadres de jambe supérieur et inférieur.

Cette technique présente l'inconvénient de fournir des coques de protection, supérieure et inférieure, fixées sur l'habillage de la coque de cuisse et de la coque tibiale. Ces coques n'étant pas mobiles, elles peuvent venir gêner l'utilisateur lorsqu'il plie ou déplie la jambe, voir provoquer un manque de confort.

Un autre inconvénient d'une telle orthèse de genou est qu'elle confère une absorption peu efficace lors d'un choc. La disposition des coques inférieure et supérieure fait que celles-ci ne protègent pas efficacement le genou.

L'invention a pour objectif de pallier les différents inconvénients de ces techniques connues.

Plus précisément, un objectif de l'invention est de fournir une orthèse de genou qui fournisse une protection fiable de la rotule du genou, quelque soit le degré de fléchissement de la jambe.

Un autre objectif de l'invention est, au moins dans un mode de réalisation en particulier, de fournir une telle orthèse qui soit simple à mettre en place et facilement adaptable à tout type de jambe.

Un objectif de l'invention est également, au moins dans un mode de réalisation en particulier, de fournir une protection du genou qui soit fiable et efficace lors d'un choc.

Encore un autre objectif de l'invention est, au moins dans un mode de réalisation en particulier, de fournir un dispositif fournissant une protection accrue.

Dans au moins un mode de réalisation particulier de l'invention, un objectif de l'invention est de fournir une telle technique, qui permette de simplifier la fabrication et le montage d'une orthèse de genou, et donc de réduire les coûts correspondants.

Ces objectifs, ainsi que d'autres qui apparaîtront plus clairement par la suite, sont atteints selon l'invention à l'aide d'une orthèse de genou comprenant :
- un cadre de jambe supérieur destiné à maintenir la cuisse, dit coque de cuisse, et un cadre de jambe inférieur destiné à maintenir le mollet, dit coque tibiale, ladite coque de cuisse et coque tibiale étant destinées à être positionnées au dessus et en dessous de la rotule du genou,
- des moyens d'articulation reliant les cadres de jambe supérieur et inférieur, destinés à être positionnés latéralement au genou,
- une coque de protection de la rotule, mobile par rapport auxdites coques de cuisse et tibiale, et destinée à être positionnée sur la rotule dudit genou, reliée auxdits moyens d'articulation par l'intermédiaire de liaisons souples.

Selon l'invention, un telle orthèse de genou comprend, outre la coque de protection de la rotule, dite première coque de protection, une seconde coque de protection apte à couvrir le jour entre ladite coque de protection de rotule et ladite coque tibiale, assujettie en mouvement à ladite coque tibiale lorsque l'orthèse est pliée et dépliée, ladite seconde coque étant de dimension supérieure à la coque de protection de la rotule et agencée par rapport à ladite première coque de sorte que ladite seconde coque est apte à glisser sur la surface extérieure de ladite première coque lorsque l'orthèse est pliée et dépliée, l'orthèse pouvant passée d'une première position dépliée dans laquelle ladite seconde coque couvre en majeure partie la première coque de protection vers une deuxième position pliée dans laquelle la seconde coque de protection couvre partiellement la première coque de protection.

Selon un aspect avantageux de l'invention, la seconde coque se trouve toujours au dessus de la première coque de protection et la coque tibiale de manière à pouvoir appuyer sur ladite première coque de protection et la coque tibiale lors d'un choc.

Préférentiellement, lesdits moyens d'articulation comprennent deux articulations polycentriques, chacune composée de deux branches, solidaires de la coque tibiale et de la coque de cuisse possédant des extrémités proximales dentées coopérant entre elles de façon à former une articulation. On obtient ainsi une articulation particulièrement bien adaptée au mouvement effectué par le genou.

Selon une approche particulièrement simple, ladite seconde coque de protection est reliée auxdits moyens d'articulation par deux lanières souples venant respectivement en prise avec deux branches respective desdites deux articulations de manière à maintenir ladite seconde coque selon un axe longitudinal de l'orthèse. Cette approche permet d'assurer un maintien en place de la seconde coque de protection fiable et efficace.

Selon l'invention, ladite coque de protection de la rotule comprend une mousse de protection sur sa surface interne, offrant un meilleur confort ainsi qu'un meilleur amorti lors d'un choc

Avantageusement, ladite coque de protection de la rotule est reliée aux moyens d'articulation par le biais de bandes pvc constituant lesdites liaisons souples.

De manière avantageuse, un guide souple est fixé sur la partie inférieure de la première coque de protection, puis sur la partie inférieure de ladite seconde coque de protection de telle façon que la seconde coque de protection reste toujours au dessus de la première coque de protection, quel que soit le degré de pliage de l'orthèse.

Selon un aspect avantageux de l'invention, ledit guide souple est une lanière de longueur sensiblement égale à la longueur de la seconde coque.

Selon un mode de réalisation, la seconde coque de protection est assujettie en mouvement à la coque tibiale par l'intermédiaire d'une bande souple joignant une extrémité de la seconde coque à la coque tibiale.

L'invention concerne également un procédé de fabrication d'une orthèse de genou dans laquelle ladite coque tibiale et ladite coque de cuisse sont fabriquées sur mesure comprenant les étapes suivantes :
- on prend les mesures de la jambe ;
- on prend l'empreinte de la jambe en appliquant une résine ;
- on moule l'orthèse à l'image de la résine.

D'autres caractéristiques et avantages de l'invention apparaîtront plus clairement à la lecture de la description suivante d'un mode de réalisation particulier de l'invention, donnée à titre de simple exemple illustratif et non limitatif, et des dessins annexés, parmi lesquels :
- la figure 1 est une vue en perspective de l'orthèse de genou selon l'invention;
- la figure 2 est une vue détaillée de la protection du genou selon l'invention en position pliée ;
- la figure 3 est une vue de l'orthèse selon l'invention en position dépliée;
- la figure 4 illustre l'articulation polycentrique équipant l'orthèse de genou selon l'invention ;
- la figure 5 est une vue en coupe de l'orthèse de genou selon l'invention, conforme à un mode de réalisation.

Comme précédemment évoqué, le principe général de l'invention repose donc sur la mise en oeuvre d'une orthèse de genou comprenant une coque de protection de la rotule.

Dans au moins un mode de réalisation de l'invention, cette nouvelle approche permet notamment de fournir une protection du genou, et en particulier de la rotule, lors d'un choc extérieur.

Par la suite, on considère une orthèse de genou équipée d'une protection du genou selon l'invention.

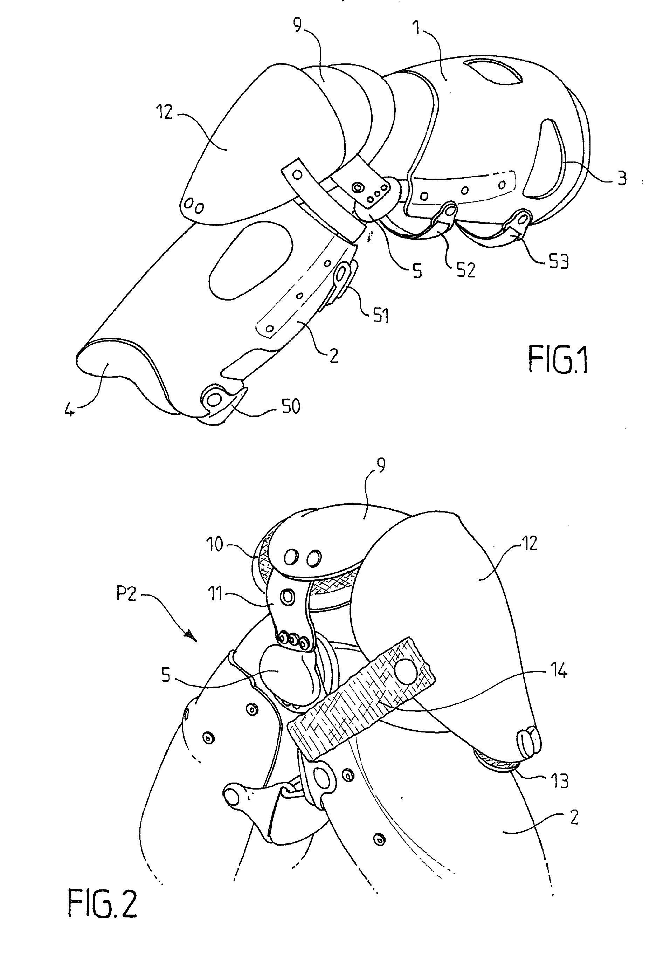

L'orthèse de genou décrite à la figure 1 comprend un cadre de jambe supérieur et inférieur 1, 2 reliés entre eux par l'intermédiaire de moyens d'articulation 5. Les cadres de jambe supérieur et inférieur 1, 2 peuvent être réalisés sur mesure et thermoformés de manière à s'adapter au mieux à la jambe de l'utilisateur.

De manière avantageuse, les cadres de jambe 1, 2 sont réalisés dans un matériau apte à résister aux chocs tel que du polyéthylène ou des matériaux composites comme la fibre de carbone, et qui soit suffisamment léger pour ne pas gêner l'utilisateur lors de la pratique de son sport.

Naturellement, tout autre matériau suffisamment solide et léger peut être utilisé pour la fabrication des cadres de jambe.

Selon un mode de réalisation de l'invention, les cadres de jambe supérieur et inférieur 1, 2 sont aptes à recevoir sur leur parois internes des coussins 3, 4 de protection adoptant la forme des cadres. Ces coussins 3, 4 peuvent être réalisés en mousse ou tout autre matériau apte à amortir un choc.

Ces coussins 3, 4 de protection peuvent être utilisés pour fournir un meilleur maintien contre la jambe de l'utilisateur.

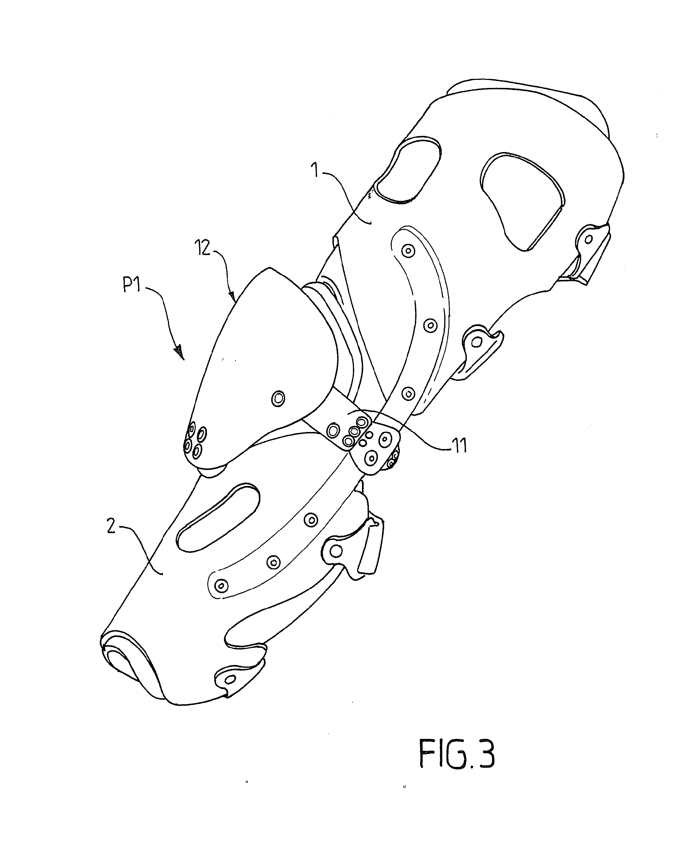

Comme illustré sur les figures 1, 2 et 3, l'orthèse de genou est maintenue sur la jambe de l'utilisateur au moyen de lanières 50 à 53.

Selon un mode de réalisation préférentiel de l'invention, les lanières 50 à 53 sont directement fixées par une de leur extrémité sur un côté des cadres inférieur ou supérieur 1, 2 et sont aptes à s'étendre sur la cuisse ou le mollet de l'utilisateur pour être fixées de l'autre côté de l'orthèse par le biais de moyens de fixation tels que du velcro disposés sur l'autre extrémité des lanières.

Des oeillets aptes à recevoir les lanières peuvent être disposés sur un côté de l'orthèse pour permettre de serrer plus ou moins les lanières 50 à 53 et ajuster la position de l'orthèse sur la jambe de l'utilisateur.

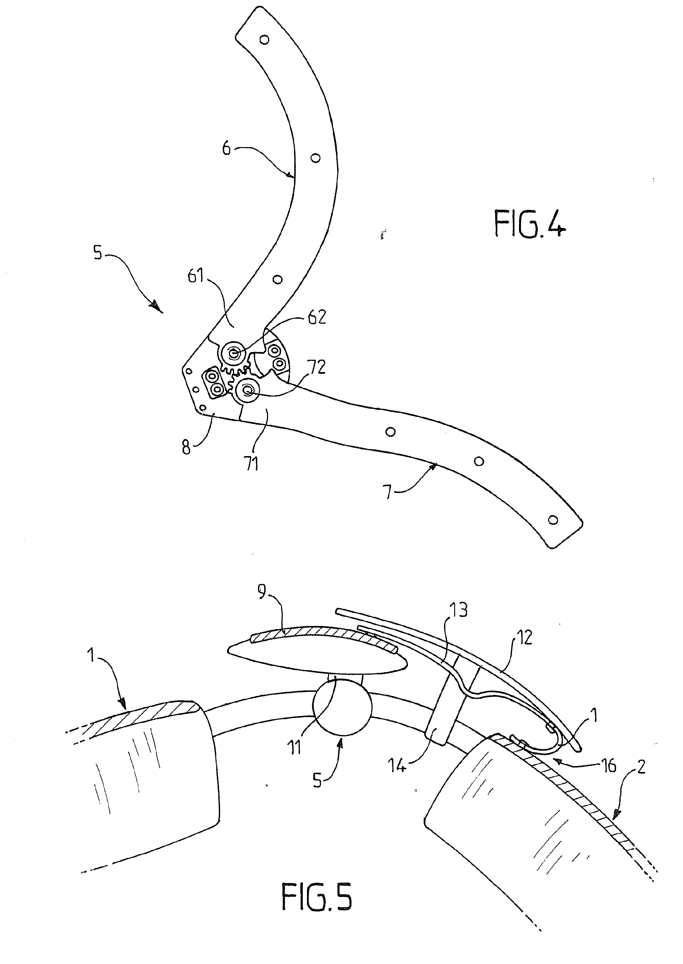

Le cadre supérieur 1 et le cadre inférieur 2 sont articulés l'un à l'autre par l'intermédiaire de moyens d'articulation 5, représentés figure 4. Les moyens d'articulation 5 comprennent deux articulations, disposées au niveau des côtés interne et externe du genou. Chaque articulation comprend une paire de branches rigides 6, 7 solidaires respectivement des coques de cuisse et tibiale, possédant des extrémités proximales dentées 61, 71 et coopérant entre elles pour former une articulation.

Les deux extrémités proximales dentées 61, 71, mutuellement engageantes sont liées en rotation sur une plaque d'articulation 8.

Les branches 6, 7 sont couplées en rotation au niveau de leurs extrémités proximales à la plaque d'articulation 8 par l'intermédiaire de pions 62, 72 servant d'axes de rotation. Un capuchon peut recouvrir les extrémités dentées 61, 71.

Avantageusement, des butées peuvent être disposées sur la plaque d'articulation 8 de façon à définir deux positions. Ces positions pouvant être la position d'extension maximale et la position de flexion maximale que le genou peut réaliser.

Chaque articulation 5, dite polycentrique, peut permettre d'avoir un centre de rotation mobile suivant le fléchissement de la jambe. En effet, les mouvements de flexion et d'extension au niveau du genou ne sont pas de simples mouvements de pivotement autour d'un axe fixe. En raison de cette complexité du mouvement du genou, l'articulation doit être adaptée en conséquence pour minimiser le risque de blessure.

La plaque d'articulation 8 est disposée latéralement au genou. Pour chaque articulation, une mousse protectrice dense peut être disposée sur la plaque 8 de façon à protéger le genou lors d'un choc.

Comme on peut le voir sur la figure 2, une coque 9 de protection de la rotule est prévue sur l'orthèse. Cette coque 9 vient se positionner au niveau de la rotule entre les coques de cuisse et tibiales 1, 2 et est reliée aux articulations polycentriques 5 disposées, de chaque côté de l'orthèse, ce qui permet de procurer à la coque 9 de protection de la rotule une certaine mobilité lorsque l'utilisateur plie la jambe. Ainsi, la rotule est constamment protégée.

La coque 9 de protection de la rotule peut être reliée aux articulations 5 au moyen de liaisons souples 11 telles que des bandes de pvc souple au nombre de deux, fixées respectivement entre la coque 9 et la plaque d'articulation 8 de chaque articulation polycentrique 5.

Selon l'invention, cette coque 9 peut être réalisée en polyéthylène et peut comporter un coussin 10 de protection de la rotule amovible pour fournir un meilleur amorti et une meilleure protection du genou lors d'un choc extérieur.

Comme illustré sur les figures, une seconde coque 12 de protection peut être disposée sur l'orthèse. Cette seconde coque 12 de protection est destinée à couvrir le jour entre la coque de protection de la rotule 9 et la coque tibiale 2 et est reliée au cadre de jambe inférieur 2 au moyen de deux bandes souples 14 disposées de chaque côté au niveau des branches de l'articulation. Ces bandes souples 14 permettent de maintenir la seconde coque 12 de protection selon l'axe longitudinal de l'orthèse de genou.

Selon un mode de réalisation de l'invention, la seconde coque 12 de protection est assujettie en mouvement à la coque tibiale 2 lorsque l'orthèse est pliée ou dépliée, notamment au moyen d'une bande souple 16 en pvc par exemple. Cette bande souple 16 relie l'extrémité de la seconde coque 12 et la coque tibiale 2.

Cette seconde coque 12 peut également être reliée à la coque 9 de protection de la rotule au moyen d'un guide souple 13.

Comme on peut le voir sur la figure 5, le guide souple 13 peut être fixée sur la coque 9 de protection de la rotule, puis sur la partie inférieure de la seconde coque 12 de protection et à la coque tibiale 2. Cet agencement particulier permet de maintenir la seconde coque 12 de protection au dessus de la coque 9 de protection de la rotule. De préférence, le guide souple 13 est une lanière de longueur sensiblement égale à la longueur de la seconde coque 12.

Selon l'exemple des figures le guide souple 13 et la bande souple 16, sont constitués par une même bande fixée à l'une de ses extrémités à la première coque 9, puis sur la partie inférieure de la seconde coque 12, et enfin à l'autre de ses extrémités, à la coque tibiale 2.

Aussi la seconde coque 12 de protection est agencée par rapport à la coque 9 de protection de la rotule de sorte que la seconde coque 12 glisse sur la surface externe de la coque de protection de la rotule lorsque l'orthèse est pliée ou dépliée.

Ainsi, lorsque l'orthèse est en position dépliée la seconde coque 12 de protection couvre en majeur partie la coque 9 de protection de la rotule, notamment entièrement, et peut passer dans une position pliée dans la quelle la seconde coque 12 de protection couvre partiellement la coque 9 de protection de la rotule, comme illustré figure 2, une partie mineure de la coque 9.

Selon l'invention, la seconde coque 12 de protection est plus grande que la coque de protection de la rotule 9 de manière à pouvoir la couvrir partiellement (position P2) ou en majeur partie (position P1) selon le fléchissement de la jambe.

Lorsque là jambe de l'utilisateur se trouve dans la position dépliée, la seconde coque 12 de protection vient couvrir le jour entre la coque tibiale 2 et la coque 9 de protection de la rotule et recouvre également en grande partie, voire entièrement, la coque 9 de protection de la rotule. Lors du passage de la position dépliée à la position pliée, la seconde coque 12 de protection assujettie en mouvement à la coque tibiale 2 vient glisser sur la coque 9 de protection de la rotule qui suit le mouvement de pivotement du genou, permettant ainsi de couvrir le jour grandissant entre la coque tibiale 2 et la coque 9 de protection de la rotule. Une fois la position de flexion maximale atteinte, la coque 9 de protection de la rotule est partiellement couverte par la seconde coque 12 de protection qui se trouve toujours au dessus de la coque 9 de protection de la rotule et au dessus de la coque tibiale 2. Quelque soit le degré de fléchissement de la jambe, la seconde coque 12 de protection peut venir appuyer sur la coque 9 de protection de la rotule et sur la coque tibiale 2, permettant de protéger de manière fiable la rotule et le tendon rotulien en particulier.

Ainsi, la seconde coque 12 se trouvant constamment au dessus, celle-ci peut transmettre un choc extérieur à la coque 9 de protection de la rotule et à la coque tibiale 2 pour absorber en partie le choc et diminuer les risques de blessure.

Naturellement, d'autres modes de réalisation auraient pu être envisagés par l'homme du métier sans pour autant sortir du cadre de l'invention définie par les revendications ci-après.

## Revendications

1. Orthèse de genou comprenant :
- un cadre de jambe supérieur (1) destiné à maintenir la cuisse, dit coque de cuisse, et un cadre de jambe inférieur (2) destiné à maintenir le mollet, dit coque tibiale, ladite coque de cuisse et coque tibiale étant destinées à être positionnées au dessus et en dessous de la rotule du genou,
- des moyens d'articulation (5) reliant les cadres de jambe supérieur et inférieur (1,2), destinés à être positionnés latéralement au genou,
- une coque (9) de protection de la rotule, mobile par rapport auxdites coques de cuisse et tibiale, et destinée à être positionnée sur la rotule dudit genou, reliée auxdits moyens d'articulation (5) par l'intermédiaire de liaisons souples (11),
**caractérisée en ce qu'**elle comprend, outre la coque (9) de protection de la rotule, dite première coque de protection, une seconde coque (12) de protection apte à couvrir le jour entre ladite coque (9) de protection de la rotule et ladite coque tibiale (2), assujettie en mouvement à ladite coque tibiale (2) lorsque l'orthèse est pliée et dépliée, ladite seconde coque (12) étant de dimension supérieure à la coque (9) de protection de la rotule et agencée par rapport à ladite première coque (9) de sorte que ladite seconde coque (12) est apte à glisser sur la surface extérieure de ladite première coque (9), lorsque l'orthèse est pliée ou dépliée, l'orthèse pouvant passée d'une première position dépliée (P1) dans laquelle ladite seconde coque (12) couvre en majeure partie la première coque (9) de protection vers une deuxième position pliée (P2) dans laquelle la seconde coque (12) de protection couvre partiellement la première coque (9) de protection.

2. Orthèse de genou selon la revendication 1 dans laquelle la seconde coque (12) se trouve toujours au dessus de la première coque (9) de protection et au dessus de la coque tibiale (2) de manière à pouvoir appuyer sur ladite première coque (9) de protection et ladite coque tibiale (2) lors d'un choc.

3. Orthèse de genou selon la revendication 1 ou 2 dans laquelle lesdits moyens d'articulation (5) comprennent deux articulations polycentriques, chacune composée de deux branches (6,7), solidaires de la coque tibiale (2) et de la coque de cuisse (1) possédant des extrémités proximales dentées coopérant entre elles de façon à former une articulation.

4. Orthèse de genou selon la revendication 3 dans laquelle ladite seconde coque (12) de protection est reliée auxdits moyens d'articulation (5) par deux lanières souples (14) venant respectivement en prise avec deux branches respectives desdites deux articulations (5) de manière à maintenir ladite seconde coque (12) selon un axe longitudinal de l'orthèse.

5. Orthèse de genou selon l'une des revendications 1 à 4 dans laquelle ladite coque (9) de protection de la rotule comprend une mousse (10) de protection sur sa surface interne.

6. Orthèse de genou selon l'une des revendications 1 à 5 dans laquelle les liaisons souples sont des bandes pvc (11).

7. Orthèse de genou selon l'une des revendications 1 à 6 dans laquelle un guide souple (13) est fixé sur la première coque (9) de protection, puis sur la partie inférieure de ladite seconde coque (12) de protection de telle façon que la seconde coque (12) de protection reste toujours au dessus de la première coque (9) de protection, quel que soit le degré de pliage de l'orthèse.

8. Orthèse de genou selon la revendication 7 dans laquelle ledit guide souple (13) est une lanière de longueur sensiblement égale à la longueur de la seconde coque (12).

9. Orthèse de genou selon l'une des revendications 1 à 8, dans laquelle la seconde coque de protection (12) est assujettie en mouvement à la coque tibiale (2) par l'intermédiaire d'une bande souple (16) joignant une extrémité de la seconde coque (12) à ladite coque tibiale (2).

10. Procédé de fabrication d'une orthèse de genou selon la revendication 1 dans laquelle ladite coque tibiale (2) et ladite coque de cuisse (1),sont fabriquées sur mesure comprenant les étapes suivantes :
- on prend les mesures de la jambe ;
- on prend l'empreinte de la jambe en appliquant une résine ;
- on moule l'orthèse à l'image de la résine.
